# EUROPEAN PATENT APPLICATION

(11) **EP 1 491 528 A1**
(43) Date of publication of application: **29.12.2004**
(21) Application number: 02707121.6
(22) Date of filing: 20.03.2002
(51) Int. Cl.: C07C 317/22, C07C 315/00

(54) **PROCESS FOR PRODUCING 4,4-BISPHENOL SULFONE**

(71) Applicant: NIPPON SODA CO., LTD., Chiyoda-ku, Tokyo 100-8165 (JP)
(72) Inventor: HIDAKA, Tomoya, c/o R & D Lab., Ichihara-shi, Chiba 290-0045 (JP)
(74) Representative: Williams, Aylsa
(86) International application number: PCT/JP2002/002702
(87) International publication number: WO 2003/078388

(57) **Abstract**

A process for producing 4,4'-bisphenol sulfone by reacting phenol with sulfuric acid, which includes conducting a dehydration reaction in a mixed solvent including an aliphatic saturated hydrocarbon having a boiling point of 175°C or higher and mesitylene, and finally distilling off the mesitylene from the reaction system, or includes conducting the reaction in a solvent including an aliphatic saturated hydrocarbon in a reaction vessel equipped with a fractionating device. Thus, 4,4'-bisphenol sulfone can be produced in high yield with a high operating efficiency. Also, provided is a process for easily producing 4,4'-bisphenol sulfone having a high purity which includes conducting the reaction in a solvent containing an aliphatic saturated hydrocarbon, subsequently adding a C₁₋₃ alcohol to the reaction mixture, separating an alcohol layer, and recrystallizing the 4,4'-bisphenol sulfone from the alcohol layer.

## Description

### Technical Field

The present invention relates to a method for producing 4,4'-bisphenol sulfone (or 4,4'-dihydroxy diphenyl sulfone, hereinafter referred to as 4,4'-BPS) by reacting phenol with sulfuric acid.

### Background Art

4,4'-BPS is a useful compound which may be used as raw material for engineering plastics, such as polysulfone (PSF) and polyether sulfone (PES). In order to produce 4,4'-BPS, methods for reacting phenol with sulfuric acid have been studied. Main problems to be solved in the methods from technical viewpoints are how efficiently water generated can be removed and how the yield of 4,4'-bisphenol sulfone (BPS) can be increased by reducing the generation of an isomeric byproduct of 2,4'-BPS.

In Japanese Unexamined Patent Application, First Publication No. Hei 3-101656, a method of producing 4,4'-BPS is disclosed in which phenol is reacted with a sulfonation agent, etc., using mesitylene as a reaction solvent, while removing water under reflux, and the reaction is proceeded while suspending 4,4'-BPS generated. Mesitylene has advantages in that it does not react with sulfonic acid, etc., and dissolves phenol very well. However, since mesitylene also dissolves 2,4'-BPS, which is a byproduct of this process, the byproduct remains in a significant amount of about 20% and it is difficult to increase the yield of 4,4'-BPS.

In Japanese Unexamined Patent Application, First Publication No. Hei 3-206073, in order to solve the above problem, a method is disclosed in which mesitylene is used and 2,4'-BPS and 4,4'-BPS are suspended in the presence of acidic catalyst so that an isomeric reaction is proceeded while evaporating the solvent. In this case, since a solid phase product is obtained in a powdery state, not in a bulk state, handling thereof becomes easy. However, since it is necessary to evaporate the solvent in order to proceed the isomeric process, the reaction condition needs to be controlled so as to adjust the rate of removing the solvent and the isomeric rate. Also, a vacuum device is required in order to completely evaporate the solvent, and it is not preferable to keep heating the products in a solid state at high temperatures after removing the solvent since it becomes a cause of coloration.

Japanese Examined Patent Application, Second Publication No. Hei 4-74347 describes a method in which phenol and sulfuric acid are reacted in dichlorobenzene or trichlorobenzene so as to selectively crystallize 4,4'-BPS, and 4,4'-BPS in a crystalline phase and 2,4'-BPS in a liquid state are eventually obtained. In this case, however, although no complicated control of the reaction is necessary as the solvent is not evaporated, the yield of 4,4'-BPS decreases because 2,4'-BPS in a liquid state remains.

In Japanese Unexamined Patent Application, First Publication No. Sho 51-98239, a method is described in which the reaction of phenol with sulfuric acid is carried out in a solvent, such as o -dichlorobenzene, and the solvent is eventually completely evaporated to produce 4,4'-BPS. According to this method, the isomeric reaction of 2,4'-BPS dissolved in the solvent to 4,4'-BPS may be proceeded by removing the solvent from the reaction system. However, there are problems in that the reaction product obtained after the evaporation of the solvent becomes a solid mass which is industrially difficult to handle, and that the control of the reaction conditions becomes necessary in order to adjust the rate of removing the solvent and the isomeric rate.

Among methods in which solvent is used, other than the above-mentioned methods, there are methods in which aliphatic hydrocarbon solvent is used (Japanese Unexamined Patent Application, First Publication No. Sho 64-9970, U.S. Patent No. 4,996,367 and so forth).

In methods using an aliphatic hydrocarbon solvent, there are advantages that conversion of 2,4'-BPS to 4,4'-BPS is proceeded without evaporating the solvent since both 4,4'-BPS and 2,4'-BPS are hardly dissolved in this solvent, and a product in a slurry state which is industrially easy to handle can be obtained.

In U.S. Patent No. 4,996,367, for example, a method is disclosed in which a hydrocarbon having a low boiling point of 70-140°C and a hydrocarbon having a high boiling point of 160-220°C are used as solvent, and a phenol layer is returned to the reaction system, which is one of at least two liquid layers obtained by distillation using the low-boiling point hydrocarbon as an azeotropic agent and then condensing the distillate. It is described that the reflux is easy since the phenol layer is the heaviest layer and becomes the lowest layer. As a preferable solvent, a combination of ISOPER (registered trademark of Exxon Chemical Company) E (initial boiling point of 115°C) and ISOPER H (initial boiling point of 176°C) is described. When the reaction system using this solvent is refluxed, there is a problem that it is not easy to return phenol and aliphatic hydrocarbon solvent to a reaction layer while removing the aqueous layer since the condensed liquid is usually separated into three layers of phenol/water/aliphatic hydrocarbon. Also, since use of two kinds of solvent of a suspending agent (for example, ISOPER (registered trademark of Exxon Chemical Company) H, initial boiling point of 176°C) and an azeotropic agent (for example, ISOPER E, initial boiling point of 115°C) is essential in the invention, there are disadvantages that the control of boiling point, etc., of the solvent becomes difficult when such solvent having different properties is recycled, and the production cost increases.

On the other hand, there have been no methods which possess advantages regarding after-treatment operation subsequent to the reaction in the above-mentioned conventional technique.

In Japanese Unexamined Patent Application, First Publication No. Sho 50-106937, a method is described in which a reaction product of phenol and concentrated sulfuric acid is dissolved in methanol aqueous solution and 4,4'-BPS is recrystallized in the methanol aqueous solution to increase the efficiency of separating 2,4'-BPS. However, this is a method used in a reaction system using no solvent, and is characterized by the use of methanol aqueous solution having significantly low concentration.

In Japanese Patent Application No. 2000-273084, a method is disclosed in which after reacting phenol and sulfuric acid in a solvent including one or more kinds of aliphatic hydrocarbon, an alcohol having a number of carbon atoms of 1-3 is added and 4,4'-BPS is recrystallized from the separated alcohol layer. Although it is described that 4,4'-BPS can be produced in a simple manner with high yield, a further improvement in the reaction efficiency is required.

Accordingly, an object of the present invention is to provide a method for producing 4,4'-BPS by reacting phenol with sulfuric acid in an aliphatic saturated hydrocarbon solvent under reflux, wherein a mixed solvent appropriate for high yield of 4,4'-BPS in a reaction process as well as subsequent processes is used to efficiently remove water, which is a byproduct, and increase the yield of 4,4'-BPS.

Also, another object of the present invention is to produce 4,4'-BPS in a simple manner with high yield by appropriately combining aftertreatment processes when an aliphatic hydrocarbon is used as a solvent.

Moreover, yet another object of the present invention is to provide methods for further improving the reaction efficiency of BPS by efficiently removing water and suppressing a distillation amount of phenol when 4,4'-BPS is produced by reacting phenol with sulfuric acid, while evaporating water, using an aliphatic hydrocarbon as a solvent.

### Disclosure of Invention

The present invention provides a process for producing 4,4'-bisphenol sulfone, including the steps of: reacting phenol with sulfuric acid under reflux in a mixed solvent including an aliphatic saturated hydrocarbon having a boiling point of 175°C or higher and a solvent capable of dissolving phenol and having a boiling point of 170°C or less while removing water, and evaporating the solvent capable of dissolving phenol and having a boiling point of 170°C or less from a reaction system. In particular, the present invention provides a process for producing 4,4'-bisphenol sulfone, wherein the solvent capable of dissolving phenol and having a boiling point of 170°C or less is mesitylene.

Also, it is preferable that the solvent capable of dissolving phenol and having a boiling point of 170°C or less be evaporated from the reaction system after the completion of a reaction.

The solvent used in the present invention is a mixed solvent including one or more kinds of aliphatic saturated hydrocarbon having a boiling point of 175°C or higher and mesitylene.

When the aliphatic saturated hydrocarbon is a mixed solvent, the distillation temperature of the mixed solvent is 175°C or higher. Examples of the solvent include ISOPER (registered trademark of Exxon Chemical Corporation) H, ISOPER L, ISOPER M, and so forth. Among these, ISOPER H (initial boiling point of 176°C) is preferable.

Examples of the solvent capable of dissolving phenol and having a boiling point of 170°C or less used in the present invention include mesitylene, chlorobenzene, etc., and mesitylene is preferable. By using the solvent which dissolves phenol, two layers of water which is distilled when dehydrated and the solvent including phenol are formed, and hence handling thereof and removal of water become significantly easy.

Although the ratio of the aliphatic saturated hydrocarbon and mesitylene in the mixed solvent is not particularly limited, the ratio may be 0.1 to 10 parts by weight, preferably 0.5 to 1.2 parts by weight, of mesitylene with respect to 1 part by weight of aliphatic saturated hydrocarbon.

It is well known that mesitylene is particularly effective, as an azeotropic agent, for removing water in a reaction of phenol with sulfuric acid to produce 4,4'-BPS. Since phenol, etc., is dissolved well when mesitylene is used as a solvent, distilled phenol and mesitylene form one layer having a specific gravity less than that of water when water is removed by distillation which is essential for a dehydration reaction. Accordingly, an operation for returning an upper layer including phenol and mesitylene to a reaction system becomes extremely easy while removing a lower and heavy aqueous layer.

However, when only mesitylene is used as a reaction solvent, the conversion of 2,4'-BPS to 4,4'-BPS is not proceeded because mesitylene dissolves 2,4'-BPS, and hence the yield decreases.

On the other hand, since an aliphatic saturated hydrocarbon solvent hardly dissolves 2,4'-isomer, the conversion of 2,4'-isomer to 4,4'-isomer can be improved and it becomes possible to obtain a large amount of 4,4'-BPS.

When a dehydration reaction is carried out under reflux using only a low boiling point aliphatic hydrocarbon solvent, it is not easy to carry out reflux operation while removing water since a condensation solution is separated in three layers of phenol / water / hydrocarbon in that order from the bottom. However, if the solvent of the present invention is used, a condensation solution is separated in two layers of water / phenol in that order from the bottom, and hence water can be efficiently removed in a dehydration reaction. As a result, it becomes possible to efficiently produce 4,4'-BPS. Also, after the dehydration reaction, the conversion reaction is enhance by evaporating mesitylene from the reaction system, and 4,4'-BPS of high purity which is suspended in the aliphatic saturated hydrocarbon can be obtained.

According to the present invention, it becomes possible to enhance removing of water and make the reflux operation easy since a solvent having a relatively low boiling point, such as mesitylene, is present in the mixed solvent used in the dehydration reaction conducted at the first half of the process, and it becomes possible to enhance the conversion reaction to 4,4'-BPS and increase the yield of 4,4'-BPS by evaporating the low boiling point solvent so that only the aliphatic saturated hydrocarbon remains as a solvent in the conversion reaction carried out at the end half of the process. Thus, it becomes possible to solve the problems associated with conventional solvent and to achieve many advantages at once by using the mixed solvent of the present invention.

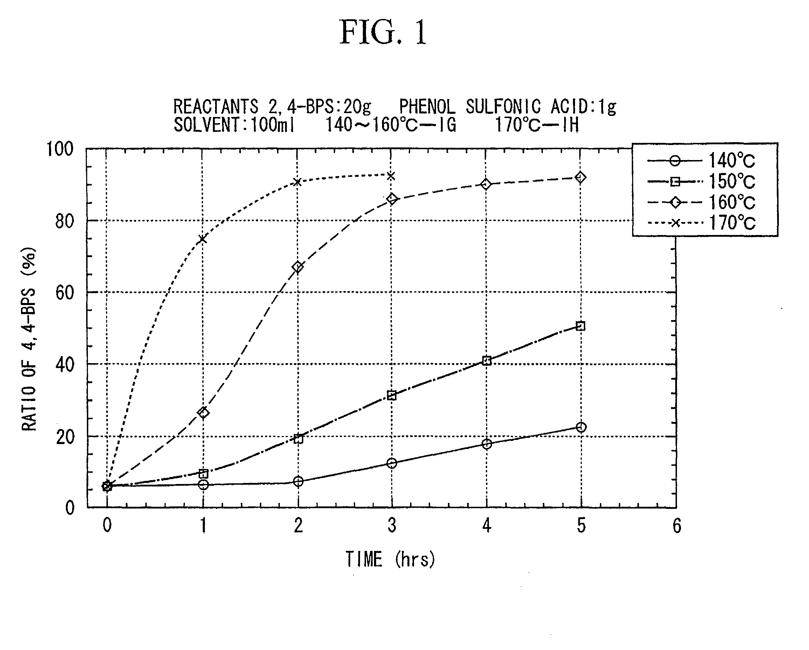

The relationship between the conversion of 2,4'-isomer to 4,4'-isomer and temperature is shown in Figure 1. Here, a mixture including (4,4'-BPS,) 2,4'-BPS and phenol sulfonic acid in the weight ratio of 20 : 1, was suspended in an ISOPER solvent, and the ratio of generating 4,4'-ismer at various temperatures and reaction time was measured using a high performance liquid chromatography (HPLC). As is obvious from the graph, the conversion rate is greatly different between 150 and 160°C, and the production of 4,4'-isomer is significantly increased. That is, the yield of 4,4'-isomer may be improved by conducting the reaction at or above this temperature.

As described above, the boiling point of the aliphatic saturated hydrocarbon is preferably higher than that temperature and significantly higher than the boiling point of mesitylene (about 162°C). In particular, it is appropriate that the boiling point of the aliphatic saturated hydrocarbon be 175°C or higher.

According to the present invention, the reaction for producing 4,4'-BPS is generally carried out as follows.

A mixed solvent including aliphatic saturated hydrocarbon and a solvent capable of dissolving phenols and having a boiling point of 170°C or less, such as mesitylene, is prepared in a reaction vessel. Then, phenol and sulfuric acid are gradually added to the mixed solvent. These may be added at the same time, or a necessary amount of phenol may be added first and then sulfuric acid may be gradually added thereto. The amount of phenol is adjusted so that the molar ratio of phenol and sulfuric acid eventually falls within the range of 2 to 3 : 1, preferably 2 : 1. The proportion of the solvent in the reaction solution, on the other hand, is adjusted so as to be 1 to 3 times, especially about 2 times, of the weight of sulfuric acid.

The reaction is carried out within the temperature range of about 145 to about 175°C.

In the reaction process, the first half thereof is occupied by a dehydration reaction in which water generated and mesitylene are subjected to an azeotropic distillation at the above-mentioned reaction temperature, and water, the solvent capable of dissolving phenols and having a boiling point of 170°C or less, such as mesitylene, and phenol are distilled off. When these are condensed, it is separated into two layers of an aqueous layer and a phenol dissolved-layer. If mesitylene is used, the aqueous layer becomes the lower layer since the phenol dissolved-layer is light, and water may be easily removed from the bottom. The distillated phenol may be continuously returned to the reaction system as a solution for a solvent.

The reflux operation is continued until it is confirmed that the dehydration reaction is completed based on the evaporation of a theoretical amount of water. Thereafter, the reflux is stopped and the low boiling point (170°C or less) solvent, such as mesitylene, is evaporated from the reaction system. The solvent having a boiling point at this range can be readily evaporated.

At that time, an additional aliphatic saturated hydrocarbon solvent, the amount of which is substantially the same as that of evaporated mesitylene may be added.

As described above, since the low boiling point solvent, such as mesitylene, is removed from the solvent, almost all of the solvent is an aliphatic saturated hydrocarbon in the end half of the reaction. In this solvent, the conversion reaction of 2,4'-BPS to 4,4'-BPS is enhanced, and the yield of 4,4'-BPS is eventually improved.

According to the operation described above, a slurry in which crystallized product is suspended in a hydrocarbon solvent is obtained. Any known methods may be used in order to recrystallize 4,4'-BPS from the solvent layer.

Also, the present invention provides a method for producing 4,4'-bisphenol sulfone by reacting phenol with sulfuric acid in a solvent including aliphatic saturated hydrocarbon, adding alcohol having a number of carbon atoms of 1-3 to the reaction solution, and recrystallizing 4,4'-bisphenol sulfone from a separated alcohol layer.

Moreover, the present invention provides a method for producing 4,4'-bisphenol sulfone by reacting phenol with sulfuric acid in a solvent including one kind or two or more kinds of aliphatic saturated hydrocarbon, adding alcohol having a number of carbon atoms of 1-3 to the reaction solution, and recrystallizing 4,4'-bisphenol sulfone from a separated alcohol layer. The above-mentioned solvent may be one kind of aliphatic saturated hydrocarbon, and it is preferable that the boiling point of the above aliphatic saturated hydrocarbon be in the range of 155 to 175°C.

According to the present invention, after 4,4'-BPS is obtained using an aliphatic saturated hydrocarbon solvent and immediately adding an alcohol to the reaction solution, an alcohol solution in which a product is dissolved is obtained by a separating operation, and it becomes possible to move onto a recrystallization process in an efficient manner. Also, the aliphatic saturated hydrocarbon may be readily recovered and recycled after the alcohol extraction operation.

According to the present invention, one kind or two or more kinds of aliphatic saturated hydrocarbon may be used as a reaction solvent. The boiling point of the aliphatic saturated hydrocarbon solvent is preferably 155°C or higher, and it is more preferably within the range of 155°C to 175°C if the solvent is constituted only by aliphatic saturated hydrocarbon. Here, when aliphatic saturated hydrocarbon forms a mixed solvent, the distillation temperature of the mixed solvent is 155°C or higher, or within the range of 155 to 175°C.

The relationship between the conversion of 2,4'-isomer to 4,4'-isomer and the temperature is shown in FIG. 1. Here, a mixture of 2,4'-BPS and phenol sulfonic acid in a weight ratio of 20 : 1 is suspended in an ISOPER solvent, and the ratio of 4,4'-isomer generated is measured at various temperatures and reaction time using a high performance liquid chromatography (HPLC). As is obvious from the graph, the conversion rate is greatly different between 150 and 160°C, and the production of 4,4'-isomer is increased as the temperature increases. That is, the yield of 4,4'-isomer may be improved by conducting the reaction at or above this temperature. Accordingly, it is preferable that the boiling point of the solvent be higher than this temperature.

On the other hand, since the boiling point of phenol is 182°C, the solvent is not refluxed in a rectification device and phenol may be refluxed instead if a reaction is carried out at a temperature higher than this temperature using a solvent having a boiling point higher than this temperature, and this is not appropriate.

The solvent of the present invention is a solvent which includes one kind or two or more kinds of aliphatic saturated hydrocarbon, and may be a solvent including only one kind of aliphatic saturated hydrocarbon or a mixed solvent including two or more kinds of aliphatic saturated hydrocarbon. Moreover, the solvent may be a mixed solvent including other solvents, such as aromatic hydrocarbon solvent, in addition to the aliphatic saturated hydrocarbon.

For the case in which a solvent constituting only aliphatic saturated hydrocarbon solvent is used, examples of the aliphatic saturated hydrocarbon solvent include nonane, decane, undecane dodecane, ISOPER (registered trademark of Exxon Chemical Corporation) G, ISOPER H, ISOPER L, ISOPER M, and a mixture thereof. It is preferable to use only one kind of these solvents. Although ISOPER solvent is a mixed solvent, it is treated as a single solvent in this specification since it can be treated as a single solvent from the viewpoint of recycling, etc., as long as it maintains a constant composition.

It is preferable that the boiling point of the solvent be 155°C or higher, in particular within the range of 155 to 175°C. Examples of a solvent having such a boiling point include nonane, decane, ISOPER G, and so forth. Among them, ISOPER G is preferable. If one kind of solvent having such a boiling point is used, it is advantageous for recycling the solvent since use of an azeotropic agent is not necessary and the solvent has a single composition.

On the other hand, when a mixed solvent of aliphatic saturated hydrocarbon and aromatic hydrocarbon solvent is used, it is preferable that the boiling point of the aliphatic saturated hydrocarbon be higher than that of the aromatic hydrocarbon solvent. Also, since the conversion process is not generally proceeded if an aromatic hydrocarbon solvent remains in a reaction system, it is desirable that the added aromatic hydrocarbon solvent be evaporated as much as possible after confirming that a theoretical amount of water is removed from the reaction system. Examples of the aliphatic saturated hydrocarbon solvent include nonane, decane, undecane, dodecane, ISOPER G, ISOPER H, ISOPER L, and ISOPER M, and examples of the aromatic hydrocarbon solvent include mesitylene, chlorobenzene, dichlorobenzene, trichlorobenzene, chlorotoluene, diethylbenzene, and xylene.

Examples of the alcohol having a number of carbon atoms of 1 to 3 used in the present invention include methanol, ethanol, propanol, and isopropanol. Among them, methanol is particularly preferable. The alcohol may be a pure product or in the form of aqueous solution.

In the present invention, the reaction of producing 4,4'-BPS is generally carried out as follows.

Aliphatic saturated hydrocarbon, which is a reaction solvent, is prepared in a reaction vessel, and phenol and sulfuric acid are gradually added thereto. At that time phenol and sulfuric acid may be added at the same time or a necessary amount of phenol may be added first and then sulfuric acid may be gradually added thereafter. The molar ratio of phenol with respect to sulfuric acid is adjusted to be within the range of 2 to 3 : 1, preferably 2 : 1.

The proportion of the reaction solvent in the reaction solution is adjusted so as to be 1 to 2 times, especially about 1.5 times, of the weight of sulfuric acid.

The reaction is carried out under reflux with stirring at a temperature in the range of about 145 to about 175°C.

The reaction is a dehydration reaction, and any known methods of dehydration may be employed as long as it is within the scope of the present invention. The dehydration is continuously carried out during the reaction. More specifically, it is easy to separate and remove water, which is azeotropically distillated, by using a device, such as a Dean and Stark.

After the completion of the dehydration reaction, the temperature at which the dehydration reaction is carried out may be maintained for a while in order to enhance the conversion reaction.

After the above reaction process is completed, the reaction solution is cooled to about 80°C. At this stage, the amount of 2,4'-BPS in the mixture of 4,4'-BPS and 2,4'-BPS is about 5% or less.

Then, 0.5 to 2 times in volume ratio, preferably about the same amount, of alcohol with respect to the reaction solvent is added to the cooled reaction solution, and the obtained mixture of 4,4'-BPS and 2,4'-BPS is dissolved therein at 65°C over about one hour. The reaction solvent of the present invention is separated in two layers since it has slight mutual solubility with alcohol. Because the product is dissolved in the alcohol layer, an alcohol solution of the product is readily obtained by this operation, and the alcohol solution may be immediately used in a recrystallization process.

If the alcohol employed is a pure product, it is possible to add water thereto. Note that it is preferable to conduct a separation operation using alcohol-water from the viewpoint of extraction efficiency, and the alcohol concentration in the alcohol-water at this state is preferably about 30 to 70% by weight.

It may be possible to add activated carbon, sodium hydroxide, etc., to the alcohol layer in order to remove impurities or adjust pH.

By adding additional water, if necessary, to the separated alcohol layer to cool the layer to about a room temperature, and then recrystallizing and drying BPS, 4,4'-BPS can be obtained.

The alcohol concentration in alcohol-water when conducting recrystallization is 10 to 50% by weight, preferably 20 to 40% by weight, and alcohol and water may be added so that alcohol-water of this concentration is eventually obtained. Since 2,4'-BPS is easily dissolved in alcohol as compared with 4,4'-BPS, the proportion of 4,4'-BPS in the crystal becomes 99% or higher.

Since the separated solvent layer including aliphatic saturated hydrocarbon does not contain products or impurities, it is readily recycled.

The present invention is also a method for producing 4,4'-bisphenol sulfone by reacting phenol with sulfuric acid in a solvent including aliphatic saturated hydrocarbon in a reaction vessel provided with a rectification device, and is also a method for producing 4,4'-bisphenol sulfone by adding, after the reaction, alcohol having a number of carbon atoms of 1 to 3 to a reaction solution and recrystallizing 4,4'-bisphenol sulfone from a separated alcohol layer.

According to the method, the reaction efficiency of BPS in the reaction of phenol with sulfuric acid may be improved by increasing the distillation amount of water and suppressing the distillation amount of phenol. Also, it becomes possible to efficiently proceed to a recrystallization process by immediately adding alcohol to a reaction solution after obtaining 4,4'-BPS using aliphatic saturated hydrocarbon solvent, so that an alcohol solution in which products are dissolved is obtained by a separating operation. Moreover, aliphatic saturated hydrocarbon may be readily recovered and recycled after the alcohol extraction operation.

In the present invention, one kind or two or more kinds of aliphatic saturated hydrocarbon may be used as a reaction solvent. The boiling point of the aliphatic saturated hydrocarbon is preferably 155°C or higher, and is more preferably within the range between 155°C and 175°C if the solvent constitutes only aliphatic saturated hydrocarbon. Here, if the aliphatic saturated hydrocarbon is a mixed solvent, the distillation temperature of the mixed solvent is 155°C or higher, and is more preferably within the range between 155°C and 175°C.

In the reaction of phenol with sulfuric acid, the conversion rate from 2,4'-isomer to 4,4'-isomer is significantly different between 150°C and 160°C, and the production of 4,4'-isomer increases as the temperature increases. That is, the yield of 4,4'-isomer may be improved by conducting the reaction at that temperature or higher. From this viewpoint, it is preferable that the boiling point of the solvent be that temperature or higher. On the other hand, since the boiling point of phenol is 182°C, the solvent is not refluxed in a rectification device and phenol may be refluxed instead if a reaction is carried out at a temperature higher than this temperature using a solvent having a boiling point higher than this temperature, and this is not appropriate.

The solvent of the present invention is a solvent which includes one kind or two or more kinds of aliphatic saturated hydrocarbon, and may be a solvent including only one kind of aliphatic saturated hydrocarbon or a mixed solvent including two or more kinds of aliphatic saturated hydrocarbon. Moreover, the solvent may be a mixed solvent including other solvents, such as aromatic hydrocarbon solvent, in addition to the aliphatic saturated hydrocarbon.

For the case where a solvent constituting only an aliphatic saturated hydrocarbon solvent is used, examples of the aliphatic saturated hydrocarbon solvent include nonane, decane, undecane dodecane, ISOPER (registered trademark of Exxon Chemical Corporation) G, ISOPER H, ISOPER L, ISOPER M, and mixtures thereof. It is preferable to use only one kind of these solvents. Although ISOPER solvent is a mixed solvent, it is treated as a single solvent in this specification since it can be treated as a single solvent from the viewpoint of recycling, etc., as long as it maintains a constant composition.

It is preferable that the boiling point of the solvent be 155°C or higher, in particular within the range of 155 to 175°C. Examples of solvents having such boiling point include nonane, decane, ISOPER G, and so forth. Among them, ISOPER G is preferable. If one kind of solvent having such a boiling point is used, it is advantageous for recycling the solvent since use of an azeotropic agent is not necessary and the solvent has a single composition.

On the other hand, when a mixed solvent including aliphatic saturated hydrocarbon and aromatic hydrocarbon solvent is used, it is preferable that the boiling point of the aliphatic saturated hydrocarbon be higher than that of the aromatic hydrocarbon solvent. Also, since the conversion process is not generally proceeded if an aromatic hydrocarbon solvent remains in a reaction system, it is desirable that the added aromatic hydrocarbon solvent be evaporated as much as possible after confirming that a theoretical amount of water is removed from the reaction system. Examples of the aliphatic saturated hydrocarbon solvent include nonane, decane, undecane, dodecane, ISOPER G, ISOPER H, ISOPER L, and ISOPER M, and examples of the aromatic hydrocarbon solvent include mesitylene, chlorobenzene, dichlorobenzene, trichlorobenzene, chlorotoluene, diethylbenzene, and xylene.

Examples of the alcohol having a number of carbon atoms of 1 to 3 used in the present invention include methanol, ethanol, propanol, and isopropanol. Among them, methanol is particularly preferable. The alcohol may be a pure product or in the form of aqueous solution.

In the present invention, the reaction of producing 4,4'-BPS is generally carried out as follows.

Aliphatic saturated hydrocarbon, which is a reaction solvent, is prepared in a reaction vessel, and phenol and sulfuric acid are gradually added thereto. At that time phenol and sulfuric acid may be added at the same time or a necessary amount of phenol may be added first and then sulfuric acid may be gradually added thereafter. The molar ratio of phenol with respect to sulfuric acid is adjusted to be within the range of 2 to 3 : 1, preferably 2 : 1.

The proportion of the reaction solvent in the reaction solution is adjusted so as to be 1 to 2 times, especially about 1.5 times, of the weight of sulfuric acid.

The reaction is carried out under reflux with stirring at a temperature in the range of about 145 to about 175°C.

The reaction is a dehydration reaction, and any known method of dehydration may be employed as long as it is within the scope of the present invention. The dehydration is continuously carried out during the reaction. More specifically, it is easy to separate and remove water, which is azeotropically distillated, by using a device, such as a Dean and Stark.

In the present invention, water may be efficiently removed from the reaction system by suppressing distillation of phenol, which is a raw material, using a reaction vessel provided with a rectification device. As a result, 4,4'-BPS can be efficiently produced. Examples of the rectification device include Widmer type rectification device, Vigrue type rectification device, Snaider type rectification device, and Hempel type rectification device.

After the completion of the dehydration reaction, the temperature at which the dehydration reaction is carried out may be maintained for a while in order to enhance the conversion reaction.

After the above reaction process is completed, the reaction solution is cooled to about 80°C. At this stage, the amount of 2,4'-BPS in the mixture of 4,4'-BPS and 2,4'-BPS is about 5% or less.

Then, 0.5 to 2 times in volume ratio, preferably about the same amount, of alcohol with respect to the reaction solvent is arbitrary added to the cooled reaction solution, and the obtained mixture of 4,4'-BPS and 2,4'-BPS is dissolved therein at 65°C over about one hour. The reaction solvent of the present invention is separated in two layers since it has slight mutual solubility with alcohol. Because the product is dissolved in the alcohol layer, an alcohol solution including the product is readily obtained by this operation and the alcohol solution may be immediately used in a recrystallization process.

If the alcohol employed is a pure product, it is possible to add water thereto. Note that it is preferable to conduct a separation operation using alcohol-water from the viewpoint of extraction efficiency, and the alcohol concentration in the alcohol-water at this state is preferably about 30 to 70% by weight.

It may be possible to add activated carbon, sodium hydroxide, etc., to the alcohol layer in order to remove impurities or adjust pH.

By adding additional water, if necessary, to the separated alcohol layer to cool the layer to about room temperature, and then recrystallizing and drying BPS, 4,4'-BPS can be obtained.

The alcohol concentration in alcohol-water when conducting recrystallization is 10 to 50% by weight, preferably 20 to 40% by weight, and alcohol and water may be added so that alcohol-water of this concentration is eventually obtained. Since 2,4'-BPS is easily dissolved in alcohol as compared with 4,4'-BPS, the proportion of 4,4'-BPS in the crystal becomes 99% or higher.

Since the separated solvent layer including aliphatic saturated hydrocarbon does not contain products or impurities, it is readily recycled.

In the meantime, when a product is produced on an industrial scale, there are occasions in which the color of a reaction mixture solution is incorporated in a crystal during a process of purifying and separating a targeted product from the reaction mixture, and it is difficult to achieve a complete decoloration even if purifying processes are repeated thereafter.

On such an occasion, although activated carbon is conventionally added to a solution of reaction mixture to facilitate decoloration of the product to some degree, complete decoloration is difficult to achieve using this method of adding activated carbon. Also, the operation is complicated since the added activated carbon must be filtered out thereafter. We performed a diligent research to find the cause of the coloration of a reaction mixture, and found that metal ions, such as iron ions, nickel ions and chromium ions, which are eluted form the reaction vessel made of SUS or contained in industrial water used as a solvent, react with phenol to form a complex and becomes a cause of coloration. Also, it was found that the coloration may be efficiently prevented by adding a chelating agent to the reaction mixture so that the metal ions are captured by the chelating agent in a process of purifying the reaction mixture and separating the targeted product.

The chelating agent is not particularly limited as long as it is a compound which binds to a metal ion and forms a chelate.

Examples of the chelating agent include compounds which are obtained by reacting a compound including an amino group, such as ammonia, polyamine and amino acids, with carbon disulfide, halogenated carboxylic acid, halogenated alcohol, etc.; salts thereof; dithiocarbamate; dimethylglyoxim; dithizone; bipyridine; phenanthroline, and so forth.

Examples of the above-mentioned polyamine include ethylenediamine, N-methyl ethylenediamine, N,N'-dimethyl ethylenediamine, 1,2-diaminopropane, 1,3-diaminopropane, diethylenetriamine, triethylenetetramine, tetramethylene pentamine, pentamethylene hexamine, and so forth.

Also, examples of the halogenated alcohol include monochloroacetate, monobromoacetic acid, monoiodeacetic acid, 1-chloropropionic acid, 2-chloropropioic acid, 1-bromopropionic acid, 2-bromopropionic acid, and so forth.

Also, examples of the halogenated alcohol include 1-chloroethanol, 2-chloroethanol, 1-bromoethanol, 2-bromoethanol, 1-chloropropanol, 2-chloropropanol, 2-chloroisopropanol, 3-chloropropanol, 1-bromopropanol, 2-bromopropanol, 2-bromoisopropanol, 3-bromopropanol, and so forth.

Examples of the chelating agent include dithiocarbamate, such as sodium dithiocarbamate and potassium dithiocarbamate; acetate derivatives of amines, such as ethylene diaminetetraacetic acid (EDTA), hydroxyethyl iminodiacetic acid (HIDA), nitrirotriacetic acid (NTA), hydroxyethyl ethylenediamine triacetic acid (HEDTA), diethylene triamine pentaacetic acid (DTHA), triethylene tetraamine hexaacetic acid (TTHA), and so forth, and salts of these acetic acid derivatives (for example, alkali metal salts of sodium, potassium, etc.; alkali earth metal salts of calcium, magnesium, etc.; ammonium salt, and so forth); ethanol amines, such as ethanol amine, N,N'-bis(2-hydroxyethyl) ethylenediamine, and N,N',N'''-tris(2-hydroxyethyl) ethylenediamine, dihydroxyethylglycine (DHEG); salts of dithiocarbamic acid derivatives of polyamines, such as sodium N,N'-bis(dithiocarboxy)ethylene diamine, potassium N,N'-bis(dithiocarboxy) ethylenediamine, sodium N,N'-bis(dithiocarboxy) trimethylene diamine, potassium N,N'-bis(dithiocarboxy) trimethylene diamine, sodium N,N'-bis(dithiocarboxy) diethylene triamine, potassium N,N'-bis(dithiocarboxy) diethylene triamine, sodium N,N',N"-tris(dithiocarboxy) diethylene triamine, potassium N,N',N"-tris(dithiocarboxy) diethylene triamine, sodium N,N'-bis(dithiocarboxy) triethylene tetraamine, potassium N,N'-bis(dithiocarboxy) triethylene tetraamine, sodium N,N',N" -tris(dithiocarboxy) triethylene tetraamine, and potassium N,N',N"-tris(dithiocarboxy) triethylene tetraamine; dimethylglyoxim, dithizone, bipyridine, phenanthrolin, and so forth.

Among these chelating agents, chelating agents which are obtained by reacting ammonia or polyamine with halogenated carboxylic acid and/or halogenated alcohol, and chelating agents (water-soluble chelating agents) which form water-soluble chelate of these salts, etc., are preferable. Use of EDTA or salts thereof are particularly preferable from the viewpoints of availability and easy-handling.

Examples of salts of EDTA include sodium EDTA2, sodium EDTA3, sodium EDTA4, potassium EDTA2, potassium EDTA3, potassium EDTA4, calcium EDTA, potassium EDTA3, diammonium EDTA, magnesium dipotassium EDTA. If a water-soluble chelating agent is added to a reaction mixture, a chelate generated is water soluble and is not incorporated into a crystal of 4,4'-bisphenol sulfone. Accordingly, a target product which is completely decolorized may be obtained.

The chelating agent may be added: 1) before or at the same time of adding alcohol and water after distillating water and phenol from a reaction mixture; 2) before separating an alcoholic aqueous layer after adding alcohol and water; 3) before adding alkali to an alcohol layer to adjust pH thereof after separating the alcoholic aqueous layer, and 4) after adjusting pH. It is preferable to add a chelating agent 4) after adjusting pH.

Examples of methods for adding a chelating agent include a method in which a chelating agent in a solid state is added to a reaction mixture, a method in which a chelating agent is dissolved in solvent, such as water, and is then added to a reaction mixture, and so forth.

The amount of chelating agent added may be properly determined based on the concentration of metal ions, such as iron ion, nickel ion, and chromium ion, contained in the reaction mixture. If the amount of chelating agent added is too small, the effect of preventing coloration by adding the chelating agent becomes insufficient. If the amount of chelating agent added is too large, on the other hand, there is a danger that excess chelating agent may contaminate the final product of 4,4'-bisphenol sulfone as an impurity although a sufficient effect of preventing coloration may be obtained. Accordingly, it is preferable to measure the concentration of metal ions contained in a reaction mixture using a known method prior to the addition of the chelating agent, and the chelating agent, the amount of which corresponds to the concentration of metal ions, be added thereto. The amount of chelating agent added is generally 0.0001 to 0.5 parts by weight, preferably 0.001 to 0.1 parts by weight, with respect to 100 parts by weight of a reaction mixture.

### BRIEF DESCRIPTION OF DRAWINGS

FIG. 1 is a graph showing a relationship between the conversion of 2,4'-isomer to 4,4'-isomer and temperature.
FIG. 2 is a graph showing a change in the distillation amount of phenol and water from a reaction solution versus time for the cases where a rectification tower is used and is not used.

### Best Mode for Carrying Out the Invention

Hereinafter, the present invention will be explained in detail with reference to examples,

### Example 1

In a four-necked flask (500 ml) provided with a stirrer, a thermometer, and water separating duct, 98.7 g (1.05 mole) of phenol, 75 ml of ISOPER H, and 50 ml of mesitylene were introduced, and 51.6 g (0.50 mole) of 95% sulfuric acid was added dropwise to the mixture with stirring at 50°C, and after that the temperature was increased.

Distillation of the reaction solution was started at about 148°C, and the distillate was separated in two layers using the water separating duct. The organic upper layer was continuously returned to the reaction system. The temperature of the reaction system reached 170°C after about four hours from the start of increasing the temperature. The generation of water is stopped, and the amount of water separated by the water separating duct was 20.5 g. After this, 50 ml of ISOPER H was added, and mesitylene was evaporated over three hours until the temperature of the reaction system reached 175°C. The composition of the reactant was analyzed using a high performance liquid chromatography (HPLC). Results were 94.7% of 4,4'-BPS, 2.8% of 2,4'-BPS, and 1.2% of phenol sulfonic acid based on the relative area ratio (measured wavelength: 231 nm).

### Comparative Example 1

In a four-necked flask (500 ml) provided with a stirrer, a thermometer, and water separating duct, 98.7 g (1.05 mole) of phenol, and 100 ml of ISOPER G were introduced, and 51.6 g (0.50 mole) of 95% sulfuric acid was added dropwise to the mixture with stirring at 50°C, and after that the temperature was increased.

Distillation of the reaction solution was started at about 144°C, and the distillate was condensed and separated in three layers including a solvent upper layer, an aqueous middle layer, and a phenol lower layer using the water separating duct. The solvent upper layer was continuously returned to the reaction system, and the phenol lower layer was extracted every 15 minutes and returned to the reaction system. The temperature of the reaction system reached 167°C after about seven hours from the start of increasing the temperature. The generation of water is stopped, and the amount of water separated by the water separating duct was 21.2 g, and the amount of phenol returned to the reaction system was 68.0 g in total. The composition of the reactant was analyzed using high performance liquid chromatography (HPLC). Results were 94.0% of 4,4'-BPS, 3.1% of 2,4'-BPS, and 1.6% of phenol sulfonic acid based on the relative area ratio (measured wavelength: 231 nm).

### Example 2

In a four-necked flask (500 ml) provided with a stirrer, a thermometer, and water separating duct, 98.7 g (1.05 mole) of phenol, and 100 ml of ISOPER G were introduced, and 51.6 g (0.50 mole) of 95% sulfuric acid was added dropwise to the mixture with stirring at 50°C, and after that the temperature was increased.

Distillation of the reaction solution was started at about 144°C, and the distillate was condensed and separated in three layers including a solvent upper layer, an aqueous middle layer, and a phenol lower layer using the water separating duct. The solvent upper layer was continuously returned to the reaction system, and the phenol lower layer was extracted every 15 minutes and returned to the reaction system. The temperature of the reaction system reached 167°C after about seven hours from the start of increasing the temperature. The generation of water stopped, and the amount of water separated by the water separating duct was 21.2 g, and the amount of phenol returned to the reaction system was 68.0 g in total. The composition of the reactant was analyzed using high performance liquid chromatography (HPLC). Results were 94.0% of 4,4'-BPS, 3.1% of 2,4'-BPS, and 1.6% of phenol sulfonic acid based on the relative area ratio (measured wavelength: 231 nm).

To the reactant, 132 ml of methanol and 103 ml of water were added and the mixture was heated to be dissolved. Then, a solvent layer (ISOPER G) was separated by a separating process. The pH of a methanol solution was adjusted to be pH 5 by adding 10% sodium hydroxide aqueous solution. After decolorization using 2.6 g of activated carbon, 103 ml of water was added to precipitate crystals, and 100.1 g (yield: 80.1%) of white crystals were obtained.

The composition of the crystal was analyzed using high performance liquid chromatography (HPLC). Results were 99.7% of 4,4'-BPS, and 0.3% of 2,4'-BPS based on the relative area ratio (measured wavelength: 231 nm).

### Example 3

In a four-necked flask (500 ml) provided with a stirrer, a thermometer, water separating duct, and Widmer rectification tube, 117.5 g (1.25 mole) of phenol, and 100 ml of ISOPER G were introduced, and 51.6 g (0.50 mole) of 95% sulfuric acid was added dropwise to the mixture with stirring at 50°C, and after that the temperature was increased.

Distillation of the reaction solution was started at about 144°C, and the distillate was condensed and separated in three layers including a solvent upper layer, an aqueous middle layer, and a phenol lower layer using the water separating duct. The solvent upper layer was continuously returned to the reaction system, and the phenol lower layer was not returned to the reaction system. The temperature of the reaction system reached 167°C after about ten hours from the start of increasing the temperature. The generation of water stopped, and the amount of water separated by the water separating duct was 20.5 g, and the amount of phenol returned to the reaction system was 21.2 g. The composition of the reactant was analyzed using high performance liquid chromatography (HPLC). Results were 95.5% of 4,4'-BPS, 3.0% of 2,4'-BPS, and 0.7% of phenol sulfonic acid based on the relative area ratio (measured wavelength: 231 nm).

The reactant was treated in the same manner as described in Example 2, and 103.2 g (yield: 82.5%) of white crystals were obtained. The composition of the crystal was analyzed using a high performance liquid chromatography (HPLC). Results were 99.6% of 4,4'-BPS, and 0.4% of 2,4'-BPS based on the relative area ratio (measured wavelength: 231 nm).

### Example 4

In a four-necked flask (300 ml) provided with a stirrer, a thermometer, water separating duct, and Widmer rectification tube, 42 g of phenol, and 42 ml of ISOPER G were introduced, and 22 g of 95% sulfuric acid was added dropwise to the mixture with stirring at 50°C, and after that the temperature was increased.

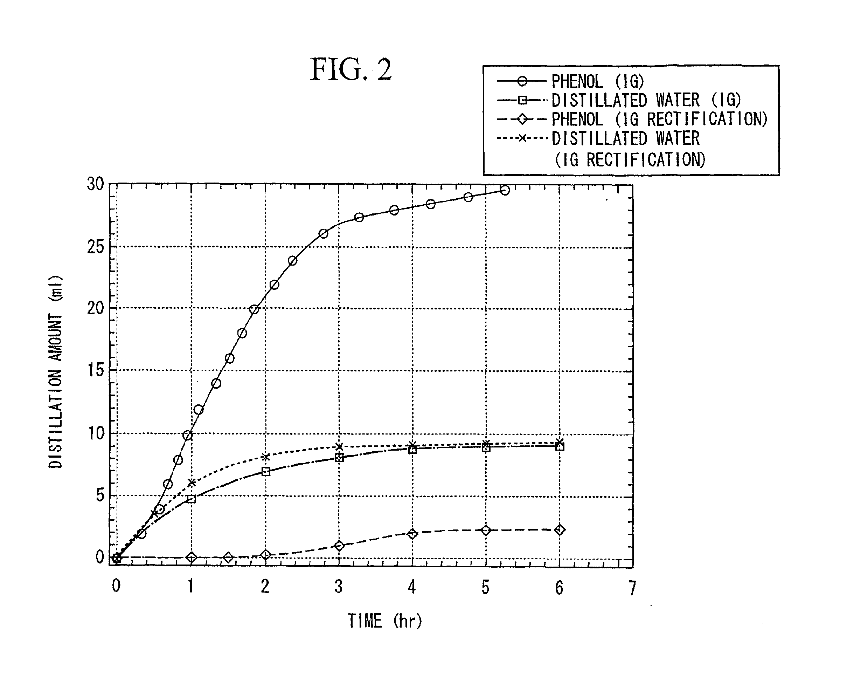

Distillation of the reaction solution was started at about 144°C, and the distillate was condensed and separated in three layers including a solvent upper layer, an aqueous middle layer, and a phenol lower layer using the water separating duct. The solvent upper layer was continuously returned to the reaction system, and the phenol lower layer was extracted every 15 minutes and returned to the reaction system. The temperature of the reaction system reached 167°C after about six hours from the start of increasing the temperature, and the generation of water stopped. The amount of water and phenol distillated in this reaction are shown in FIG. 2. The amount of phenol distillated by the end of the reaction and returned to the reaction system was 2.2 ml in cumulative total, and the amount of water removed from the reaction system was 9.1 ml. The composition of the reactant was analyzed using high performance liquid chromatography (HPLC). Results were 90.8% of 4,4'-BPS, 3.4% of 2,4'-BPS, 1.3% of phenol sulfonic acid, and 2.1% of trimer based on the relative area ratio (measured wavelength: 231 nm).

To the reactant, 56 ml of methanol and 44 ml of water were added and the mixture was heated to be dissolved. Then, a solvent layer (ISOPER G) was separated by a separating process. The pH of a methanol solution was adjusted to be pH 5 by adding 10% sodium hydroxide aqueous solution. After decolorization using 1.1 g of activated carbon, 60 ml of water was added to precipitate crystals, and 46.2 g (yield: 86.8%) of white crystals were obtained.

The composition of the crystal was analyzed using high performance liquid chromatography (HPLC). Results were 99.5% of 4,4'-BPS, and 0.4% of 2,4'-BPS based on the relative area ratio (measured wavelength: 231 nm).

### Comparative Example 2

The same operation as described in Example 4 was repeated except that the four necked flask was not provided with the Widmer rectification tube. The amount of water and phenol which were distillated during the synthetic reaction of BPS are shown in FIG. 2. The amount of phenol distillated by the end of the reaction and returned to the reaction system was 30 ml in cumulative total, and the amount of water removed from the reaction system was 9.0 ml. To the reactant, methanol and water were added and the same operation as in Example 4 was conducted to obtain 41.2 g (yield: 77.4%) of white crystals. The composition of the crystal was analyzed using high performance liquid chromatography (HPLC). Results were 99.5% of 4,4'-BPS, and 0.4% of 2,4'-BPS based on the relative area ratio (measured wavelength: 231 nm).

### Reference Example

In a four-necked flask (500 ml) provided with a stirrer, a thermometer, and water separating duct, 131.6 g (1.4 mole) of phenol and 70 ml of ISOPER G were introduced, and 72.2 g (0.7 mole) of 95% sulfuric acid was added dropwise to the mixture with stirring at 50°C. After the completion of dropwise addition, the temperature of the reaction system was gradually increased while stirring the reaction mixture. Distillation of the reaction solution was started at about 144°C, and the distillate was condensed and separated in three layers including a solvent upper layer, an aqueous middle layer, and a phenol lower layer using the water separating duct. The solvent upper layer was continuously returned to the reaction system, and the phenol lower layer was extracted every 15 minutes and returned to the reaction system. The temperature of the reaction system reached 165°C after about seven hours from the start of increasing the temperature, and the generation of water stopped. Nearly all water and unreacted phenol were evaporated from the reaction solution.

Then, the reaction mixture was cooled to 80°C, and 185 ml of methanol was added. The solution was stirred at 65°C for one hour. Then, 144 ml of water was added for separating layers, and an alcoholic aqueous layer was obtained.

After this, 10% sodium hydroxide aqueous solution was added to adjust pH to 5, and then 1 ml of 5% aqueous solution of sodium EDTA2 was added dropwise. After sufficiently stirring the alcoholic aqueous layer, the solution was cooled to 30°C for precipitation. Precipitated crystals were filtered and dried to obtain 140.2 g (yield: 80%) of a targeted product of 4,4'-dihydroxydiphenyl sulfone powder.

Purity of the obtained 4,4'-dihydroxydiphenyl sulfone was 99.5% or higher. The b value of the product measured by a color-difference meter (type: 1001DP, a product of Nippon Denshoku Co., Ltd.) was 2.5 or less, and no coloration was observed.

### Industrial Applicability

As described above, the method of the present invention is characterized by using a mixed solvent including one kind or two or more kinds of aliphatic saturated hydrocarbon having a boiling point of 175°C or higher and a solvent capable of dissolving phenol and having a boiling point of 170°C or less, such as mesitylene. Since the low boiling point solvent, such as mesitylene, functions as an azeotropic agent and also dissolve phenol, two layers are formed by distillation during a dehydration process. Accordingly, removal of water during the dehydration reaction is carried out with extreme efficiency, and hence it becomes easy to conduct a reflux operation. Also, since the low boiling point solvent, such as mesitylene, is evaporated after the completion of the dehydration reaction, it becomes possible to improve the yield of 4,4'-BPS by enhancing the conversion reaction in an aliphatic saturated hydrocarbon solvent. According to the present invention, it becomes possible to achieve many advantages described above by using one kind of mixed solvent.

Moreover, it becomes possible to produce 4,4'-BPS with high yield in a form of a slurry which is easy to handle. Since the aliphatic saturated hydrocarbon solvent of the present invention is insoluble with methanol, a product may be efficiently extracted from the reaction solution using methanol, and hence may be efficiently transferred to a subsequent recrystalization process. Furthermore, it is possible to recycle the separated solvent layer. In addition, when one kind of solvent having a boiling point within the specific scope of the present invention is used, it is not necessary to use an azeotropic agent and the solvent may be recycled.

Also, for the case where a rectifying tube is employed, a distillation amount of phenol is significantly reduced and water, which is a byproduct, may be efficiently removed. Therefore, it also becomes possible to improve the reaction efficiency.

## Claims

1. A process for producing 4,4'-bisphenol sulfone, comprising the steps of: reacting phenol with sulfuric acid in a mixed solvent including an aliphatic saturated hydrocarbon having a boiling point of 175°C or higher and a solvent capable of dissolving phenol and having a boiling point of 170°C or less under reflux while removing water, and evaporating the solvent capable of dissolving phenol and having a boiling point of 170°C or less from a reaction system.

2. A process for producing 4,4'-bisphenol sulfone according to claim 1, wherein the solvent capable of dissolving phenol and having a boiling point of 170°C or less is mesitylene.

3. A process for producing 4,4'-bisphenol sulfone, comprising the steps of: after reacting phenol with sulfuric acid in a solvent including an aliphatic saturated hydrocarbon, adding an alcohol having a number of carbon atoms of 1 to 3 to an obtained reaction solution; and recrystallizing 4,4'-bisphenol sulfone from a separated alcohol layer.

4. A process for producing 4,4'-bisphenol sulfone, comprising the step of: reacting phenol with sulfuric acid in a solvent including an aliphatic saturated hydrocarbon using a reaction vessel provided with a rectifying device.

5. A process for producing 4,4'-bisphenol sulfone, comprising the steps of: reacting phenol with sulfuric acid in a solvent including an aliphatic saturated hydrocarbon using a reaction vessel provided with a rectifying device; adding an alcohol having a number of carbon atoms of 1 to 3 to an obtained reaction solution; and recrystallizing 4,4'-bisphenol sulfone from a separated alcohol layer.
